# EUROPEAN PATENT SPECIFICATION

(11) **EP 1 370 564 B1**
(45) Date of publication and mention of the grant of the patent: **26.01.2005**
(21) Application number: 02722200.9
(22) Date of filing: 11.03.2002
(51) Int. Cl.: C07F 9/10, C07C 39/21, A61K 31/05, A61K 31/685, A61P 3/00, A61K 7/40

(54) **RESVERATROL-PHOSPHOLIPIDS COMPLEXES**
RESVERATROL-PHOSPHOLIPIDE KOMPLEXE
COMPLEXES RESVERATROL-PHOSPHOLIPIDES

(30) Priority: 13.03.2001 IT MI20010528
(43) Date of publication of application: 17.12.2003
(73) Proprietor: Istituto Biochimico Pavese Pharma SPA, 27100 Pavia (IT)
(72) Inventor: PIFFERI, Giorgio, I-20117 Milan (IT); ANZAGHI, Piergiorgio, I-20078 San Colombano Al Lambro (IT); STEFLI, Rosanna, I-27100 Pavia (IT)
(74) Representative: Gervasi, Gemma, Dr.
(86) International application number: PCT/EP2002/002641
(87) International publication number: WO 2002/072591

(56) References cited:
- EP-A- 0 275 224
- EP-A- 1 064 931
- WO-A-01/30336
- WO-A-99/04747

## Description

### Field of the invention

The present invention refers to the preparation of new complexes obtained by reacting natural or synthetic phospholipids with resveratrol and the use thereof in pharmaceutical and cosmetic fields.

### Prior art

Resveratrol is a well known stilbene-structured phenolic compound in *cis* and *trans* forms, also as glycosidic esters thereof.

Resveratrol is mainly produced by plants as a natural antifungal agent to counter parasite attacks. Its natural sources are Vitis vinifera, pine tree, eucalyptus, Liliaceae, Polygonaceae and Leguminosae and many vegetable species included

in daily diet, such as grapes, peanuts, and pine-kernels.

In popular Japanese and Chinese medicine, resveratrol is the active ingredient of the Polygonum cuspidatum powdered root, used in the treatment of dermatitis, atherosclerosis, hyperlipidemia and inflammatory diseases (H. Arichi *et al*., Chem. Pharm. Bull., 30, 1766-1770, 1982). Recent researches have identified resveratrol's considerable antioxidant and radical blocking effects *in vivo* and *in vitro* and its pharmacological actions of potential interest for the treatment of some cardiovascular conditions.

Further studies *in vitro* and *in vivo* have established that *trans*-resveratrol can inhibit platelet aggregation and reduce plasma lipid levels, which are the main factors responsible for deaths caused by cardiovascular diseases (H. Arichi *et al*., Chem. Pharm. Bull., 30, 1766-1770, 1982; C.R. Pace-Asciak *et al*., Clin. Chim. Acta, 235, 207-219, 1995; E.N. Frankel, Lancet, 341, 1103-4, 1993). Other studies have found that resveratrol is an antitumoral and anti-inflammatory agent because it inhibits the arachidonic cascade and the successive formation of prostaglandins (M. Jang et al., Science, 275, 218, 1997; Y. Kimura *et al*., Biochim. Biophys. Acta, 834, 275-8, 1985).

Resveratrol is potentially interesting from a therapeutic and cosmetic stand point because it slows down the ageing and cellular degeneration processes.

WO9904747 discloses a skin care composition comprising resveratrol in an amount of from 0.00002 to 10wt.% and a cosmetically acceptable vehicle.

EP 1064931 describes a composition comprising in a physiologically acceptable medium in particular suitable for topical and mucosal application at least on e hydroxystilbene and ascorbic acid or analogue thereof.

WO0130336 discloses a method for preventing or treating a skin condition, disorder or disease that is responsive to resveratrol comprising administering to the susceptible or affected area of the individual's skin a therapeutically effective amount of a topical pharmaceutical formulation that comprises a topical carrier and an active agent selected from resveratrol, pharmacologically acceptable salts and esters, amides, prodrugs and analogues thereof and combination of any of the foregoing.

Resveratrol is mainly present in wine and grapes in the *trans* form and relating glycosylate derivatives. In red wine the total resveratrol concentration ranges from 0.6 to 11 mg/l, depending on the place of origin and it is higher than in the white and rosé types because, in the manufacturing process of red wine, the grape must is fermented with marc, while prolonged soaking naturally extracts resveratrol from grape skins and seeds. The daily consumption of two to five glasses of red wine provides plasma Resveratrol levels, that are sufficient to produce antiplatelet and hypolipidemic effects.

In the glycosidic form, resveratrol is more soluble, but less active and less lipophilic. Aglycon is more active, but practically insoluble and, therefore, scarcely bioavailable.

In any case, Resveratrol is absorbed thanks to the solvent power of ethanol contained in wine. However physicians advise against consuming prolongedly wine in order to avoid liver and central nervous system damage. Therefore, the compound, when isolated, is poorly bioavailable.

EP 275224 describes phospholipidic complexes of flavonoid extracts of *Vitis vinifera* and their application in therapeutic and cosmetic topical compositions.

### Description of the invention

It is an object of the present invention to provide a bioavailable resveratrol-phospholipids complex, which exhibits increased lipophilia and, consequently, favours gastrointestinal absorption.

The phospholipids selected to this end are either natural (vegetable or animal origin) or synthetic. According to the present invention phospholipids may be used having different titre. Preferably phospholipids are used having a titre comprised between 45 to 98%.

The acylic chain of these products consist of linoleic, palmitic, oleic, linolenic, stearic, and gamma-linoleic acids.

Preferred phospholipids used for preparing the complex according to the present invention are soya or egg phosphatidylcholine, di-stearoyl-phosphatidylcholine, di-palmitoyl-phosphatidylcholine, phosphatidylethanolamine, phosphatidylserine. Resveratrol in the complex according to the present invention is preferably present as aglycon and in this case the complex is characterised by the following formula (I): 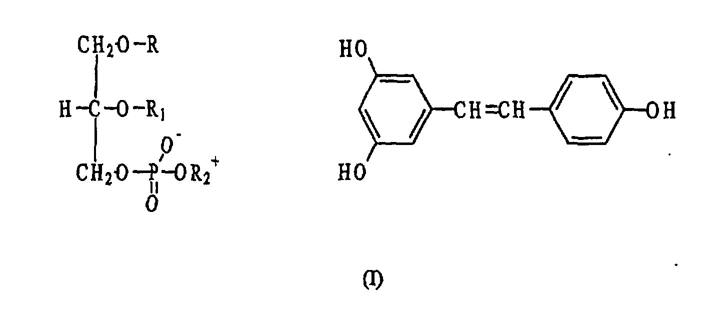 wherein R and R₁ equal to or different from each other represent the acyl radical of palmitic, stearic, oleic, linoleic, linolenic acid and R₂ is selected from the group consisting of: -CH₂-CH₂-N⁺(CH₃)₃, -CH₂-CH₂-N⁺H₃, -CH₂-CH-(COOH)-N⁺H₃. Resveratrol in the complexes according to the present invention is preferably in the trans form.

The complex according to the present invention is formed with a process comprising reacting Resveratrol (1 mole) in an aprotic polar solvent, preferably acetone or dioxane or in an apolar solvent preferably methylene chloride, with from 1 to 2 moles of phospholipids and recovering said product preferably by concentrating or precipitating said complex with a hydrocarbon solvent, e.g. hexane.

In the complex, the phosphatidylcholine polar terminations interact chemically, whereas the non-polar portion consisting of long-chain fatty acids residues is not bound and, therefore, the complex becomes highly liposoluble. The complex exhibits peculiar physicochemical characteristics (high solubility in fats and organic solvents, lower melting point) and modified IR and NMR spectra.

The aforementioned complexes exhibit a high diffusibility through artificial and natural membranes and, consequently, may be better orally absorbed.

The antioxidant activity of Resveratrol and the complex was determined with Miller-C. Rice-Evans' method (N.J.Miller, C.Rice-Evans, Redox Rep.161-171, 1996).

The chromogen substance ABTS [2,2'-azinobis(3-ethyl-benzothiazolin-6-sulfonate] is converted in the presence of potassium persulfate into the corresponding monocationic radicalic form ABTS⁺ showing a blue green colour. The addition of an antioxidant analogue of Vitamin E and denominated Trolox causes, proportionally to its concentration, a decoloration of the solution, whose absorbance value is read on the spectrophotometer at 734nm.The antioxidant activity (TAA) of a sample is determined by the comparison of the absorbance value of the radicalic solution on contact with Trolox and on contact with the sample to be tested, and it is expressed as Trolox concentration (mM). A Resveratrol solution 1mM (0.228mg/ml) and an ethanol solution of the complex of example 1 (1.042mg/ml) corresponding to 1 mM Resveratrol concentration were subjected to the text to determine the antioxidant power of Resveratrol and verify that the complex maintains the same antioxidant activity as Resveratrol.

The results are reported in the following Table I.

**Table I**

| Compound | TAA (mM Trolox) |
|---|---|
| Trans Resveratrol | 2.4 |
| Complex of Example 1 | 2.4 |

As it results from the above data the *in vitro* antioxidant activity of the complex remains equal to that of Resveratrol as such. The complex permeability through a natural membrane was compared with that of *trans*-Resveratrol on the rabbit colon by the "Ussing Chamber's" method. Said method proved a good correlation with the *in vivo* situation. It follows that a forecast of the complex transport is possible across the gastrointestinal barrier.

This device consists of: 6 cells divided into two parts by a septum; which accommodate the tissue obtained from the rabbit colon; an oxygenation system; a system for the circulation of a specific buffer solution maintained at constant temperature by heating; and finally electrodes for measuring the compound concentration variations, wherefrom the *apparent permeability coefficient* (Papp), expressed in cm/sec, is obtained. The permeability coefficient of the complex as per Example 1, compared with that of *trans*-Resveratrol, was determined by the "Ussing Chamber".

The results obtained are shown in Table I.

**Table II**

| Compound | Papp in the colon (x 10⁻⁶ cm/sec) | % absorbed fraction *in vivo* |
|---|---|---|
| *trans*-Resveratrol | 0.20 | 2 |
| Complex of Ex. 1 | 1.6 | 23 |

A pharmacokinetic study was conducted by administering orally to 5 rats the complex as in Example 1 and to 5 rats equimolecular doses of *trans*-Resveratrol. The product concentration in blood was determined at prefixed time intervals. The data obtained are shown in Table III.

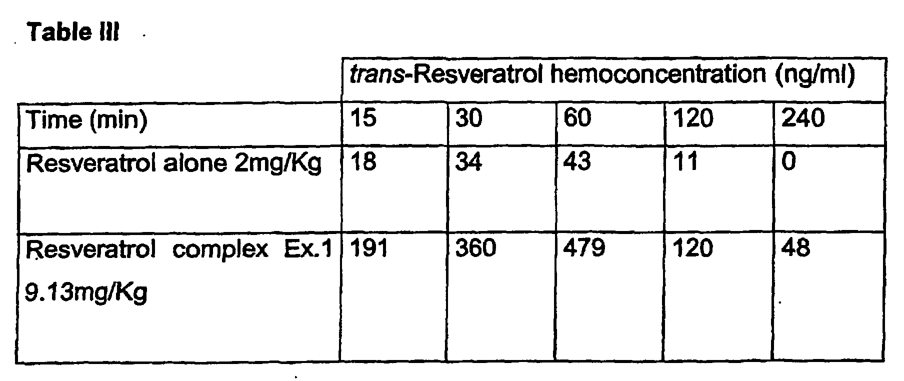

As may be seen, the bioavailability of the complex is much higher.

The platelet antiaggregating activity of the complex as per Example 1 was determined *in vitro* (C.R. Pace-Asciak *et al*., Clin. Chim. Acta, 235, 207-219, 1995) on a sample of plasma having a known platelet concentration, by measuring the difference in optical density detected by thrombin addition (to induce aggregation) in the presence or in the absence of the complex.

The complex at a concentration of 1.3 µM can inhibit platelet aggregation by 8%. Therefore the present invention further relates to a pharmaceutical composition containing as the active ingredient at least one complex according to the present invention in combination with suitable excipients and /or diluents.

The pharmaceutical composition according to the present invention is in particular characterised by having antioxidant and radical blocking activity.

The pharmaceutical composition is preferably suitable to be orally administered and more preferably in the form of tablets, capsules, granules.

A further subject of the present invention is a cosmetic composition containing at least one complex according to the present invention as the active ingredient to be used in particular for slowing down the ageing and cell degeneration processes.

The cosmetic composition according to the present invention is preferably in the form of cream, gel , ointment and emulsion.

A further subject of the present invention relates to a free flowing powder containing at least one complex according to the present invention in association with at least one excipient selected from silica, starch, talc, microcrystalline cellulose, lactose in an amount ranging from 10 to 50% by weight, based on the total free flowing powder weight which in particular may be used as a preformulated mixture for the preparation of the oral pharmaceutical compositions according to the present invention.

The following examples are reported of the present invention for illustrative, but not limitative purposes.

### Example 1 - Complex trans-Resveratrol-soya phosphatidylcholine 1:1

228.25 mg (1mmole) *trans*-Resveratrol and 813.95 mg phosphatidylcholine (titre 94.6% 1 mmole) were dissolved in 20 ml hot anhydrous acetone under stirring for 3 hrs. The resulting solution was poured in 30 ml *n*-hexane and maintained at room temperature for 18 - 24 hrs. The gelatinous residue was decanted washed with hexane, filtered and dried in an oven under vacuum. 1020 mg (yield 97.87%) were obtained of a waxy product soluble in chloroform.

The IR spectrum of said product clearly differs from that of Resveratrol for the disappearance at 3200 cm⁻¹ of the band of the phenolic hydroxy groups stretching replaced by the corresponding bands at 2080 and 2680 cm⁻¹.

In H-NMR spectrum the signals (chemical shift δ) result shifted towards lower values, if compared to those of Resveratrol as such.

From said NMR data it also results that the intermolecular bonds are caused by the interaction between hydroxy groups of Resveratrol and phosphatidylcholine and that said product is a complex.

### Example 2 - Complex trans-Resveratrol-soya phosphatidylcholine 1:1.2

381 mg (1.67mmoles) *trans*-Resveratrol and 1571 mg phosphatidylcholine (titre: 98%; 2mmoles) were dissolved in 20 ml hot anhydrous acetone, and stirred under reflux for about 1 hr. The resulting yellow ochre solution was concentrated, poured in 30 ml *n*-hexane and maintained at room temperature overnight. The doughy precipitate was decanted, washed and crystallised with hexane, filtered and dried in an oven under vacuum at 40°C for 4 hrs. 1886 mg (yield 96.65%) of a product were obtained, as a yellow powder soluble in chloroform.

IR and NMR spectra, confirm also in this case the complex formation.

### Example 3 - Complex trans-Resveratrol-soya phosphatidylcholine 1:1.5

114 mg *trans*-Resveratrol (0.5 mmoles) and 1283 mg phosphatidylcholine (titre: 45%, 0.75 mmoles) were dissolved in 20 ml hot anhydrous acetone and stirred under reflux for about 1 hr. The resulting solution was concentrated in a rotary flask under vacuum until a waxy residue was obtained. Said residue was crystallised with *n*-hexane, filtered and dried in an oven under vacuum at 35°C. 1263 mg (yield 90.4%) were recovered of a yellow ochre and oily powder, soluble in chloroform.

From IR and NMR spectra the product results to be a complex with intermolecular bonds between the hydroxy groups of Resveratrol and Phosphatidylcholine.

### Example 4 - Complex trans-Resveratrol di-stearoyl phosphatidylcholine 1:1

228.25 mg (1mmole) *trans*-Resveratrol and 894 mg distearoylphosphatidylcholine (titre 97%; 1mmole) were dissolved in 10 ml hot anhydrous acetone. The resulting yellow ochre solution was stirred for 3 hrs, concentrated and dried in an oven under vacuum at 35°C for 4 hrs. 1064 mg of a product were recovered as a yellow powder soluble in chloroform.

IR and NMR spectra confirm that the product is a complex.

### Example 5 - Complex trans-Resveratrol-dipalmitoylphosphatidylcholine 1:1.2

228.25 mg (1mmole) *trans*-Resveratrol were dissolved in 10 ml dioxane and further added with a dioxane solution containing 969.6 mg dipalmitoylphosphatidylcholine (titre: 99%, 1.2 mmoles). The solution was stirred at room temperature for 3 hrs and liophylised. 1134 mg (yield 94.6%) were obtained of a light yellow-beige liophylised product, soluble in chloroform. IR and NMR spectra confirm that the product is a complex.

### Example 6 -Complex trans Resveratrol-phosphatidylethanolamine 1:2

114 mg (0.5mmoles) *trans*-Resveratrol were dissolved in 10 ml hot acetone and poured in 10 ml acetone containing 1450 mg phosphatidylethanolamine (titre 50%; 1 mmole).

The yellow ochre solution thus obtained was maintained under stirring for 3 hrs, concentrated , and poured in 15 ml n-hexane and maintained at room temperature overnight. The waxy precipitate was crystallised with n-hexane, filtered and dried in an oven under vacuum at 35°C for 4 hrs.1486 mg (yield 95%) were obtained as a yellow powder soluble in chloroform. IR and NMR spectra confirm the formation of a complex.

### Example 7 - trans-Resveratrol-soya phosphatidylserine complex (1:2)

114 mg (0.5mmoles) *trans*-Resveratrol and 788 mg phosphatidylserine (titre: 98%;1mmole) were dissolved in 30 ml methylene chloride and left under stirring for 3 hrs. The resulting solution was concentrated, poured in 15 ml *n*-hexane and maintained at room temperature overnight. The doughy precipitate obtained was dried in an oven under vacuum at 40°C for 4 hrs. 850 mg (yield 94.2%) of a product were obtained, as a yellow powder soluble in chloroform.

IR spectrum evidences the disappearance of the typical bands of Resveratrol and NMR spectroscopy evidences that the peaks typical of Resveratrol and phosphatidylserine result shifted, if compared to the corresponding NMR peaks of Resveratrol as such and phospatidylserine as such, demonstrating the formation of a complex.

### Example 8 -Preformulated product for the preparation of solid oral formulations

The complex of example 1 was dissolved in acetone and added with at least one excipient such as silica, lactose, starch, talc, microcrystalline cellulose in total amounts ranging from 10 to 50% by weight based on the total free mixture weight. The solvent was removed by evaporation under vacuum. The product thus obtained was sieved through a 60 mesh screen, thereby recovering a free-flowing powder.

### Example 9 - Tablets

Each tablet weighing 100 mg contains:

| | |
|---|---|
| Complex of Example 1 | 4.6 mg |
| Starch | 30 mg |
| Lactose | 40 mg |
| Silica | 10 mg |
| Talc | 5 mg |
| Magnesium stearate | 2.5 mg |

These tablets were prepared using a manual press, with a concave punch of 8mm diameter.

### Example 10- Capsules

Capsules were prepared, each containing 4.6 mg complex as per Example 1, (equal to 1 mg Resveratrol), 32 mg starch, 45 mg lactose, 20 mg silica, 2.5 mg magnesium stearate.

### Example 11 - Emulsion

100 g of an emulsion containing 0.22% Resveratrol were prepared by mixing 1 g of the complex as per Example 1 with 70 g demineralised water, 6 g mineral oil, 5 g glycerin, 4 g PEG 150 stearate, 3 g propylene glycol, 3 g cetearyl alcohol, 3 g polysorbate 60, 3 g cetyl alcohol, 2 g carbomer, and 0.15 g sodium methyl parahydroxybenzoate.

### Example 12-gel

A gel containing 4% Resveratrol was obtained by mixing 20% by weight of the Complex of Example 1, 15% ethanol, 65% water, carbopol, triethanolamine, glycerol, lavender essence.

### Example 13- soft capsules

Capsules of soft gelatine were prepared according to known technique containing an oily solution consisting of from 3 to 7 mg of the complex of example 1 in 350 to 750 mg of a vegetable or animal oil in particular oenothera , borage , fish oil or another oil enriched in γ-linolenic acid.

## Claims

1. Complex of Resveratrol with a phospholipid.

2. The complex as claimed in claim 1 having the general formula (I): 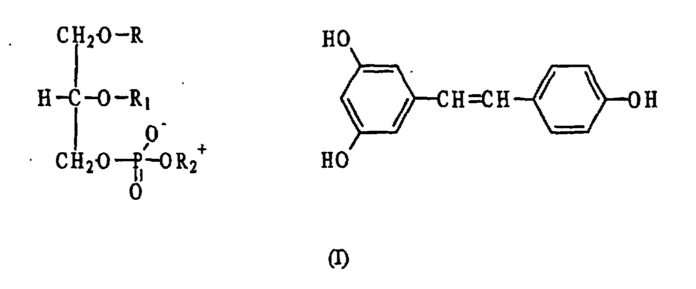 wherein R and R₁ equal to or different from each other represent the acyl radical of palmitic, stearic, oleic, linoleic, linolenic acid and R₂ is selected from the group consisting of: -CH₂-CH₂-N⁺(CH₃)₃, -CH₂-CH₂-N⁺H₃, -CH₂-CH-(COOH)-N⁺H₃.

3. The complex as claimed in claim 2, wherein Resveratrol is in the *trans* form

4. The complexes as claimed in any of claims 1-3, wherein the phospholipid is of synthetic or natural origin.

5. The complex as claimed in claim 4 wherein said phospholipid of natural or synthetic origin is selected from the group consisting of: phosphatidyl choline, distearoylphosphatidylcholine, phosphatidylethanolamine and phosphatidylserine.

6. The complex as claimed in claim 4 wherein phosphatidylcholine comes from soya or from egg.

7. The complex as claimed in any of claims 4-6, wherein the starting phospholipid has a titre ranging from 45 to 98%.

8. The complexes as claimed in any of claims 1-7 wherein Resveratrol and phospholipid are in a molar ratio of 1:1 to 1:2.

9. A process for the preparation of the complex as claimed in any of claims 1 to 8, comprising reacting 1 mole Resveratrol in an aprotic polar or an apolar solvent with from 1 to 2 moles phospholipid and separating the resulting complex.

10. The process as claimed in claim 9, wherein said solvent is selected from the class consisting of acetone and dioxane, methylene chloride.

11. A pharmaceutical composition containing at least one Resveratrol complex as claimed in any of claims 1 to 8 as the active ingredient, in combination with suitable excipients and/or diluents.

12. The pharmaceutical composition as claimed in claim 11 having antioxidant and radical blocking activity.

13. The pharmaceutical composition as claimed in any of claims 11 and 12 suitable to be orally administered in the form of tablets, capsules, granules.

14. The pharmaceutical composition according to claim 13 in the form a soft capsule containing from 3 to 7 mg of the complex according to anyone of claims 1-8 with from 350 to 750 mg of an oil of animal or vegetable origin enriched in γ-linolenic acid.

15. The pharmaceutical composition according to claim 14 wherein said oil is selected from the group consisting of oenothera , borage , fish oil.

16. A free flowing powder comprising at least one complex as claimed in any of claims 1-8 and at least one excipient selected from the group consisting of: silica, starch, talc, microcrystalline cellulose in an amount ranging from 10 to 50% by weight, based on the total free flowing powder weight.

17. Use of the free flowing powder as claimed in claim 16, as a preformulated mixture for the preparation of the oral pharmaceutical compositions as claimed in claim 13.

18. A cosmetic composition containing at least one complex as claimed in any of claims 1 to 8 in combination with suitable excipients and/or diluents.

19. The cosmetic composition as claimed in claim 18 suitable for slowing down the ageing and cell degeneration processes.

20. The cosmetic composition as claimed in any of claims 18 and 19 suitable for topical administration in the form of cream, gel, ointment and emulsion.

## Patentansprüche

1. Komplex von Resveratrol mit einem Phospholipid.

2. Komplex nach Anspruch 1 mit der allgemeinen Formel (I): 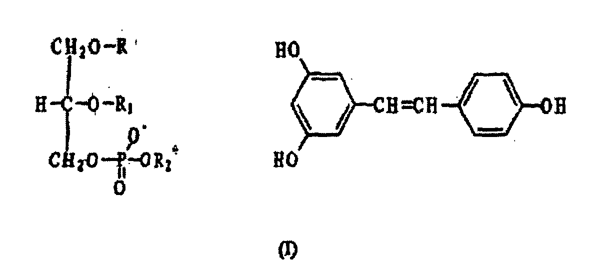 wobei R und R₁, identisch oder von einander verschieden, den Acylrest von Palmitinsäure, Stearinsäure, Ölsäure, Linolsäure oder Linolensäure darstellt und R₂ ausgewählt wird aus der Gruppe bestehend aus: -CH₂-CH₂-N⁺(CH₃)₃, -CH₂-CH₂-N⁺H₃, -CH₂-CH-(COOH)-N⁺H₃.

3. Komplex nach Anspruch 2, wobei Resveratrol in trans-Form vorliegt.

4. Komplexe nach einem der Ansprüche 1 - 3, wobei das Phospholipid synthetischen oder natürlichen Ursprungs ist.

5. Komplex nach Anspruch 4, wobei genanntes Phospholipid mit natürlichem oder synthetischem Ursprung ausgewählt wird aus der Gruppe bestehend aus: Phosphatidylcholin, Distearoylphosphatidylcholin, Phosphatidylethanolamin und Phosphatidylserin.

6. Komplex nach Anspruch 4, wobei Phosphatidylcholin aus Soja oder aus Ei stammt.

7. Komplex nach einem der Ansprüche 4 - 6, wobei das Ausgangsphospholipid einen Titer zwischen 45 und 98 % aufweist.

8. Komplexe nach einem der Ansprüche 1 - 7, wobei Resveratrol und Phospholipid in einem molaren Verhältnis von 1 : 1 bis 1 : 2 vorliegen.

9. Verfahren zur Herstellung des Komplexes nach einem der Ansprüche 1 - 8, umfassend das Zur-Reaktion-Bringen von 1 mol Resveratrol in einem aprotischen polaren oder einem unpolaren Lösungsmittel mit 1 bis 2 mol Phospholipid und Abtrennung des erhaltenen Komplexes.

10. Verfahren nach Anspruch 9, wobei genanntes Lösungsmittel ausgewählt wird aus der Klasse bestehend aus Aceton, Dioxan und Methylenchlorid.

11. Pharmazeutische Zusammensetzung, enthaltend mindestens einen Resveratrolkomplex nach einem der Ansprüche 1 - 8 als wirksamen Bestandteil, in Kombination mit geeigneten Hilfsstoffen und/oder Verdünnungsmitteln.

12. Pharmazeutische Zusammensetzung nach Anspruch 11 mit antioxidativer und Radikal-blockierender Wirkung.

13. Pharmazeutische Zusammensetzung nach Anspruch 11 oder 12, geeignet für eine orale Verabreichung in Form von Tabletten, Kapseln oder Granulaten.

14. Pharmazeutische Zusammensetzung nach Anspruch 13 in Form einer weichen Kapsel mit 3 bis 7 mg des Komplexes nach einem der Ansprüche 1 - 8 mit 350 bis 750 mg eines tierischen oder pflanzlichen Öls, angereichert mit γ-Linolensäure.

15. Pharmazeutische Zusammensetzung nach Anspruch 14, wobei genanntes Öl ausgewählt wird aus der Gruppe bestehend aus Oenothera, Borretsch und Fischöl.

16. Rieselfähiges Pulver, umfassend mindestens einen Komplex nach einem der Ansprüche 1 - 8 und mindestens einem Hilfsstoff ausgewählt aus der Gruppe bestehend aus: Siliciumoxid, Stärke, Talkum und mikrokristalliner Cellulose, in einer Menge zwischen 10 und 50 Gew.-%, bezogen auf das Gesamtgewicht des rieselfähigen Pulvers.

17. Verwendung des rieselfähigen Pulvers nach Anspruch 16 als eine vorformulierte Mischung zur Herstellung der oralen pharmazeutischen Zusammensetzung nach Anspruch 13.

18. Kosmetische Zusammensetzung, enthaltend mindestens einen Komplex nach einem der Ansprüche 1 - 8 in Kombination mit geeigneten Hilfsstoffen und/oder Verdünnungsmitteln.

19. Kosmetische Zusammensetzung nach Anspruch 18, geeignet für die Verlangsamung des Alterungs- und Zelldegenerationsprozesses.

20. Kosmetische Zusammensetzung nach einem der Ansprüche 18 und 19, geeignet für die topische Verabreichung in Form einer Creme, eines Gels, einer Salbe und einer Emulsion.

## Revendications

1. Complexe de Resvératrol avec un phospholipide.

2. Complexe tel que revendiqué à la revendication 1 ayant la formule générale (I): 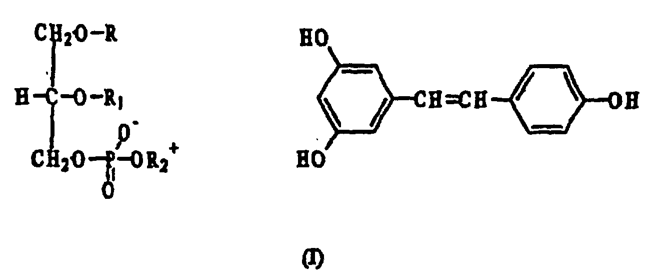 où R et R₁, égaux ou différents l'un de l'autre, représentent le radical acyle de l'acide palmitique, stéarique, oléique, linoléique, linolénique et R₂ est sélectionné dans le groupe consistant en: -CH₂-CH₂-N⁺(CH₃)₃, -CH₂-CH₂-N⁺H₃, -CH₂-CH- (COOH) -N⁺H₃.

3. Complexe tel que revendiqué à la revendication 2, où le Resvératrol est sous forme *trans*.

4. Complexes tels que revendiqués dans l'une quelconque des revendications 1-3, où le phospholipide est d'origine synthétique ou naturelle.

5. Complexe tel que revendiqué à la revendication 4, où ledit phospholipide d'origine naturelle ou synthétique est sélectionné dans le groupe consistant en: phosphatidyl choline, distéaroylphosphatidylcholine, phosphatidyl éthanolamine et phosphatidylsérine.

6. Complexe tel que revendiqué à la revendication 4, où la phosphatidylcholine provient du soja ou de l'oeuf.

7. Complexe tel que revendiqué dans l'une quelconque des revendications 4-6, où le phospholipide de départ a un titre compris entre 45 et 98 %.

8. Complexes tels que revendiqués dans l'une quelconque des revendications 1-7, où le Resvératrol et le phospholipide sont à un rapport molaire de 1:1 à 1:2.

9. Procédé pour la préparation du complexe tel que revendiqué dans l'une quelconque des revendications 1 à 8, comprenant la réaction de 1 mole de Resvératrol dans un solvant aprotique polaire ou apolaire avec 1 à 2 moles du phospholipide et séparation du complexe résultant.

10. Procédé tel que revendiqué à la revendication 9, où ledit solvant est sélectionné dans la classe consistant en acétone et dioxane, chlorure de méthylène.

11. Composition pharmaceutique contenant au moins un complexe de Resvératrol tel que revendiqué dans l'une quelconque des revendications 1 à 8, en tant qu'ingrédient actif, en combinaison avec des excipients et/ou diluants appropriés.

12. Composition pharmaceutique telle que revendiquée à la revendication 11 ayant une activité anti-oxydante ou de blocage des radicaux.

13. Composition pharmaceutique telle que revendiquée dans l'une quelconque des revendications 11 et 12 appropriée pour être administrée oralement sous la forme de comprimés, capsules, granules.

14. Composition pharmaceutique selon la revendication 13 sous la forme d'une capsule molle contenant 3 à 7 mg du complexe selon l'une quelconque des revendications 1-8 avec 350 à 750 mg d'une huile d'origine animale ou végétale enrichie en acide γ-linolénique.

15. Composition pharmaceutique selon la revendication 14 où ladite huile est sélectionnée dans le groupe consistant en huile d'oenothère, bourrache ou poisson.

16. Poudre fluide comprenant au moins un complexe tel que revendiqué dans l'une quelconque des revendications 1-8 et au moins un excipient sélectionné dans le groupe consistant en: silice, amidon, talc, cellulose microcristalline en une quantité allant de 10 à 50 % en poids, en se basant sur le poids total de la poudre fluide.

17. Utilisation de la poudre fluide selon la revendication 16 en tant que mélange préformulé pour la préparation des compositions pharmaceutiques orales telles que revendiquées à la revendication 13.

18. Composition cosmétique contenant au moins un complexe tel que revendiqué dans l'une quelconque des revendications 1 à 8 en combinaison avec des excipients et/ou diluants appropriés.

19. Composition cosmétique telle que revendiquée à la revendication 18 appropriée pour ralentir les processus de vieillissement et dégénérescence cellulaire.

20. Composition cosmétique telle que revendiquée dans l'une quelconque des revendications 18 et 19 appropriée pour administration topique sous la forme de crème, gel, onguent et émulsion.
